# EUROPÄISCHE PATENTSCHRIFT

(11) **EP 1 588 678 B1**
(45) Veröffentlichungstag und Bekanntmachung des Hinweises auf die Patenterteilung: **13.06.2007**
(21) Anmeldenummer: 05007935.9
(22) Anmeldetag: 12.04.2005
(51) Int. Cl.: A61F 5/01

(54) **Modulare Hüftorthese**
Modular hip orthosis
Orthèse de hanche modulaire

(30) Priorität: 23.04.2004 DE 102004020980
(43) Veröffentlichungstag der Anmeldung: 26.10.2005
(73) Patentinhaber: Wilhelm Julius Teufel GmbH, 73117 Wangen (DE)
(72) Erfinder: Weigel, Jochen, 73650 Winterbach (DE); Anker, Torsten, 71554 Weissach (DE)
(74) Vertreter: Patentanwälte Ruff, Wilhelm, Beier, Dauster & Partner

(56) Entgegenhaltungen:
- DE-A1- 4 222 601
- US-A- 3 902 482
- US-A- 4 144 881
- US-A- 4 393 542
- US-A- 6 039 707

## Beschreibung

### Anwendungsgebiet und Stand der Technik

Die Erfindung betrifft eine Hüftorthese zum Anlegen im Bereich der Hüfte und eines Oberschenkels eines Anwenders mit einem Orthesenteil, bestehend aus einem Beckenring, einem Femur-Teil, einem Condylenteil und Verbindungsmitteln mit einem Gelenk, wobei die Verbindungsmittel den Beckenring mit dem Condylenteil verbinden, wobei das Femur-Teil entweder zwischen dem Beckenring und dem Condylenteil mit den Verbindungsmitteln verbunden ist oder ein Bestandteil der Verbindungsmittel ist, und wobei das Gelenk ein Beugen des Hüftgelenks ermöglicht.

Zur orthopädietechnischen Behandlung eines Hüftgelenks, insbesondere in Bezug auf Führung und Stabilisierung, sind verschiedene Orthesen und Bandagen bekannt. Diese Orthesen und Bandagen werden insbesondere dann angewandt, wenn eine erhöhte Hüftgelenkluxationsneigung oder Hüftgelenkinstabilitäten bei einem Patienten zu erwarten sind bzw. vorliegen.

Hüftgelenksbandagen zeichnen sich dabei durch eine gute Passform und einer hohen Akzeptanz bei den Patienten aus. Sie wirken durch eine Kompression auf den Weichteilmantel und stabilisieren das Hüftgelenk durch diese Kompression der Weichteile. Als negativ an Hüftgelenksbandagen wird allerdings angesehen, dass sie den Hüftgelenkkopf nur mit geringer Kraft in die Hüftgelenkpfanne drücken und dadurch eine Luxationsgefahr bestehen bleibt. Darüber hinaus beschränken sie den Patienten in zu geringem Maße in seiner Bewegungsfreiheit, so dass dieser seinen Oberschenkel und das Hüftgelenk in unter orthopädischen Gesichtspunkten nicht wünschenswerte Stellungen bringen kann.

Eine Alternative zu Hüftgelenksbandagen stellen die eingangs genannten Hüftgelenksorthesen dar. Bei diesen wird über ein aus mehreren Komponenten bestehendes Gerüst der Oberschenkel in seiner Bewegung geführt. Durch ein verstellbares sehr rigides Gelenk wird der Oberschenkel in eine Abduktionsstellung gebracht und dadurch der Hüftgelenkkopf besser in die Hüftgelenkpfanne eingestellt. Zusätzlich wird die Beugung im Hüftgelenk durch einen Anschlag begrenzt. Bei anderen bekannten Hüftorthesen wird durch ein sogenanntes Dreipunktprinzip eine Kraft mittels eines Femur-Teils auf den Oberschenkel ausgeübt wobei das Femur-Teil über Verbindungsmittel mit einem im Bereich der Hüfte oder der Taille angeordneten Gürtel und mit einem über dem Knie angeordneten Condylenteil verbunden ist, so dass die Kraft auf den Oberschenkel von diesen Komponenten aufgebracht werden kann. Die auf den Oberschenkel ausgeübte Kraft drückt den Hüftgelenkkopf in die Hüftgelenkpfanne und verhindert so eine Luxation des Gelenks. Die Verbindungsmittel, welche den Taillen- bzw. Hüftring mit dem Condylenteil verbinden, weisen ein Gelenk auf Höhe des Hüftgelenks auf, welches eine Beugung des Oberschenkels ermöglicht. Als nachteilig an diesen Hüftgelenksorthesen wird angesehen, dass die Passform aufgrund der starren Bauteile oft unbefriedigend ist und dass solche Hüftgelenksorthesen in aufwändiger Art und Weise für den Patienten angepasst werden müssen. Außerdem sind Orthesen dieser Art sehr schwer und unförmig, so dass sie nicht unter der Kleidung des Patienten getragen werden können. Die Akzeptanz ist daher bei vielen Patienten nicht sehr hoch. Der gravierendste Nachteil ist jedoch, dass sie nur so lange befriedigend funktionieren, solang die Achse des Hüftgelenks und die Achse des Gelenks der Hüftorthese koaxial zueinander sind. Wenn die Hüftgelenksorthese nicht ideal angepasst ist oder nicht ideal sitzt, kommt es beim Bewegungsablauf des Patienten zu starken Belastungen des Hüftgelenks, welche eine Luxation des Hüftgelenks begünstigen können.

Die US-PS 4 144 881 zeigt ein Gelenk für verschiedene orthopädische Vorrichtungen, welches aus mehreren Teilgelenken aufgebaut ist. Die Teilgelenke sind kettenartig miteinander verbunden und können jeweils bezüglich ihres maximalen Auslenkwinkels eingestellt werden.

Die. US-PS 6 039 707 zeigt eine Hüftorthese, die über ein Taillenband und über ein Oberschenkelband verfügt, an denen jeweils Verbindungsträger angebracht sind, die miteinander über ein Gelenk auf Höhe des Hüftgelenks verbunden sind. Dieses Gelenk ist mit einer Spiralfeder ausgestattet, so dass der schenkelseitige Verbindungsträger nach vorne vorgespannt ist und dadurch eine Beugung des Hüftgelenks begünstigt. Die Verbindungsträger sind taillen- und schenkelseitig jeweils verstellbar ausgebildet. Ebenso ist das Taillenband verstellbar ausgebildet und verfügt über ein Aluminiumteil, das den Körper ungefähr zur Hälfte umgibt.

### Aufgabe und Lösung

Der Erfindung liegt die Aufgabe zugrunde, eine Hüftorthese zur Verfügung zu stellen, die einer Luxation wirksam entgegenwirkt und den Anwender bzgl. seiner Bewegungsfreiheit nur in unter orthopädischen Gesichtspunkten sinnvoller Art und Weise einschränkt.

Gelöst wird diese Aufgabe durch eine Hüftorthese der eingangs genannten Art, wobei das Gelenk mehrere Gelenkachsen aufweist, die gemeinsam eine Bewegungsfläche für eine Bewegung des Condylenteils und des Femur-Druckteils relativ zum Beckenring definieren, wobei das Gelenk in eine Richtung senkrecht zur Bewegungsfläche bzw. parallel zu einer Gelenkachse federnd ausgebildet ist. Vorteilhafte sowie bevorzugte Ausgestaltungen der Erfindung sind in den weiteren Ansprüchen enthalten und werden im folgenden näher erläutert. Der Wortlaut der Ansprüche wird durch ausdrückliche Bezugnahme zum Inhalt der Beschreibung gemacht.

Der Beckenring ist dabei so geartet, dass er einen sicheren und bequemen Halt am Becken des Anwenders aufweist. Zweckmäßig ist beispielsweise ein Beckenring, der aus einem biegbaren Material gefertigt ist, so dass er für Anwender mit verschiedenen Körpermaßen geeignet ist. Das Condylenteil dient der Aufnahme des Oberschenkels unmittelbar oberhalb des Knies. Zweckmäßigerweise weist es eine U-förmige Form, die den Oberschenkel von vorne und seitlich teilweise umschließt, sowie Mittel auf, die einen festen Halt gewährleisten, beispielsweise in Form eines Verschlussriemens, der je nach Umfang des Oberschenkels verschieden weit zugezogen werden kann. Die Verbindungsmittel bestehen beispielsweise aus einer oder mehreren Schienen, wobei es sich vorzugsweise um Metallschienen handelt. Diese Schienen verbinden den Beckenring mit dem Condylenteil und erlauben eine Befestigung des Femur-Druckteils. Das Femur-Druckteil übt von außen Druck auf den Oberschenkel aus und hält so den Hüftgelenkkopf in der Hüftgelenkpfanne. Die vom Oberschenkel auf das Femur-Druckteil ausgeübte Gegenkraft wird durch den Beckenring und das Condylenteil kompensiert. Neben einer Befestigung des Femur-Druckteils an Verbindungsmitteln zwischen dem Beckenring und dem Condylenteil, die aus nur einer Schiene bestehen, gibt es auch die Möglichkeit, zwei Schienen als Verbindungsmittel zu verwenden, die beide mit dem Femur-Druckteil verbunden sind. Bei einer solchen Ausgestaltung kann das Femur-Druckteil lösbar mit einer der beiden oder beiden Verbindungsschienen verbunden sein, so dass eine Verstellbarkeit hinsichtlich der Länge der Verbindungsmittel am Femur-Druckteil gegeben ist. Das Gelenk gestattet ein Beugen des Oberschenkels, wobei es die Bewegung insofern begrenzt, als dass diese nur in einer unter orthopädischen Gesichtspunkten wünschenswerten Ebene möglich ist. Die Verwendung mehrerer Gelenkachsen verhindert eine schädliche Inkongruenz, bei der die Achse des Hüftgelenks und die Achse des Gelenks der Verbindungsmittel nicht miteinander übereinstimmen. In einem solchen Fall der Inkongruenz würde sich eine Beugung des Oberschenkels in starken Kräften niederschlagen, die auf das Hüftgelenk wirken und eine Luxation begünstigen könnten. Die Verwendung eines Gelenks mit mehreren Gelenkachsen führt dazu, dass das Gelenk sich automatisch so ausrichtet, dass die Schwenkachse des Gelenks übereinstimmt mit der Gelenkachse des Hüftgelenks.

In einer Weiterbildung der Erfindung ist das Gelenk ein Kettengelenk mit im wesentlichen parallelen Gelenkachsen, wobei die Anzahl der Achsen mindestens zwei und vorzugsweise drei ist. Ein solches Gelenk ist fertigungstechnisch einfach herzustellen und weist eine hohe Stabilität auf. Durch die parallelen Gelenkachsen ist gewährleistet, dass eine Bewegung des Gelenks nur in einer Ebene erfolgen kann. Der wesentliche Vorteil eines solchen Kettengelenks, der darin liegt, dass sich automatisch eine Kongruenz zwischen der Gelenkachse des Hüftgelenks und der Gelenkachse der Orthese einstellt, wird schon bei einem Kettengelenk mit zwei Gelenken erreicht. Ein Kettengelenk mit drei Gelenken ermöglicht darüber hinaus eine Längenanpassung der Verbindungsmittel, so dass verhindert wird, dass das Femur-Druckteil und das Condylenteil sich relativ zum Oberschenkel bewegen, wenn der Oberschenkel gebeugt wird.

In einer Weiterbildung der Erfindung begrenzt das Gelenk ein Beugen des Oberschenkels in einer oder beiden Richtungen bei einem jeweils festgelegten Winkel, nach vorne vorzugsweise bei ca. 60° bis 80°, insbesondere bei 70°. Auf diese Art und Weise wird erreicht, dass keine Beugestellung möglich ist, die eine Luxation des Hüftgelenks begünstigt. Eine Begrenzung des Winkels für eine Bewegung nach hinten ist üblicherweise nicht erforderlich. Wenn es jedoch zu einer sehr weiten Beugung des Oberschenkels nach vorne kommt, kann der Hüftgelenkkopf anatomisch bedingt leichter aus der Hüftgelenkpfanne herausgleiten. Eine solche Stellung kann durch eine erfindungsgemäße Winkelbegrenzung verhindert werden.

Biegbar ausgebildete Verbindungsmittel erlauben dem Anwender, trotz der Wirkung, die von dem festen Gestell der Hüftorthese ausgeht, eine Abduktion und Adduktion des Beines. Dies ist besonders deshalb wünschenswert, da auf diese Art und Weise wieder eine normale Gehbewegung des Anwenders möglich ist, bei der die Gesamtmasse des Körpers des Anwenders wechselnd vollständig auf jeweils einem Bein lastet. Durch die Adduktion des Beines ist es dem Anwender möglich, das Bein bei den Gehbewegungen unmittelbar unter dem Schwerpunkt des Körpers zu positionieren, so dass eine natürlich Gehbewegung möglich ist. Dies ist insbesondere im Hinblick auf eine ausgewogene Belastung der Muskeln des Anwenders wünschenswert.

In einer Weiterbildung der Erfindung sind die Verbindungsmittel mit dem Beckenring so verbunden, dass der Oberschenkel von der natürlichen Stellung des Oberschenkels nach außen gedrückt wird, vorzugsweise mit einer Kraft von 10 bis 30 Newton und vorzugsweise bis zu einem Winkel von 10° bis 30°. Durch eine solche Ausrichtung der Verbindungsmittel wird erreicht, dass in einer entspannten Grundstellung der Hüftgelenkkopf in die einer anatomisch vorteilhaften Position zur Hüftgelenkpfanne eingestellt wird. Dies vermindert die Gefahr einer Luxation weiter, da der Anwender nur mit entsprechendem Kraftaufwand das Bein in die ungünstige Adduktionsstellung bringen kann. Der Winkel, unter dem die Verbindungsmittel mit dem Beckenring verbunden sind, ist vorzugsweise einstellbar. Dies kann beispielsweise mittels kleiner Keilelemente erreicht werden, die zwischen Verbindungsmitteln und Beckenring eingesetzt werden. Durch die Auswahl und Ausrichtung dieser Keilelemente kann so gezielt die gewünschte Abduktionsstellung erreicht werden.

In einer Weiterbildung der Erfindung ist das Bandagenteil mit dem Orthesenteil vorzugsweise lösbar verbunden und so ausgebildet, dass es zumindest einen Teil der Hüfte und des Oberschenkels umschließt.

Eine solche Hüftorthese vereinigt die Vorteile einer Bandage mit den Vorteilen einer stabilen Orthese. So wird durch den Bandagenteil eine Kompressionswirkung auf die Weichteile ausgeübt, die zu einer Stabilisierung des Hüftgelenkskopfs in der Hüftgelenkpfanne führt. Der Orthesenteil stabilisiert das Bein so, dass es zu keiner Luxation kommt. Das Orthesenteil und das Bandagenteil sind vorzugsweise voneinander trennbar, so dass sie separat ausgetauscht werden können. Das Orthesenteil kann so ausgebildet sein, dass es mit und ohne das Bandagenteil verwendbar ist.

In einer Weiterbildung der Erfindung ist ein Oberschenkelsegment des Bandagenteils, insbesondere ein Oberschenkelsegment an der Innenseite des Oberschenkels, lösbar mit der übrigen Bandage verbunden, vorzugsweise mittels eines Reißverschlusses und/oder eines Klettverschlusses. Auf diese Art und Weise kann ein derartiges Segment des Bandagenteils separat vom Hauptteil der Bandage getrennt werden. Dies ist zweckmäßig, da gerade die Teile der Bandage, die an der Innenseite des Oberschenkels anliegen, unter hygienischen Gesichtspunkten besonderen Belastungen ausgesetzt sind. Eine separate Trennbarkeit ermöglicht es, diese Teile häufiger auszuwechseln oder getrennt von dem Hauptteil der Bandage zu waschen. Durch möglichst einfache Verbindungsmittel mit dem Hauptteil der Bandage, wie beispielsweise einem Reißverschluss und/oder einem Klettverschluss, ist es möglich, dass auch Menschen, die in ihrer Bewegungsfreiheit eingeschränkt sind, dieses Segment der Bandage problemlos entfernen und austauschen können.

In Weiterbildung der Erfindung kann eine Hüftorthese der eingangs genannten Art mit Anpassbarkeit an verschiedene Anwender vorgesehen werden, die ermöglicht, dass die Verbindungsmittel bezüglich ihrer Länge änderbar sind, dass die Verbindungsmittel bezüglich ihrer Verbindungsstelle mit dem Beckenring verstellbar sind und/oder dass der Beckenring und das Condylenteil aus flexiblem Material sind, beispielsweise Thermoplast, und mittels elastischem oder verstellbaren Verschlussmittel an verschiedene Becken- bzw. Knieumfänge anpassbar sind.

Eine derart ausgestaltete Hüftorthese kann an verschiedene Anwender angepasst werden, ohne dass dafür Teile ausgetauscht werden müssen. Des weiteren ist es auch nicht erforderlich, für Anwender verschiedener Statur verschiedene Hüftorthesen vorzusehen, da alle wesentlichen Unterscheidungsmerkmale zwischen verschiedenen Anwendern durch ein solches System individuell berücksichtigt werden können. Als Verbindungsmittel kann beispielsweise ein System mit zwei Metallschienen dienen, die ineinander verschiebbar und miteinander fixierbar sind, so dass den Unterschieden im Abstand zwischen Becken und Knie verschiedener Anwender Rechnung getragen werden kann. Zweckmäßig ist auch ein System mit zwei Metallschienen, die beide mit dem Femur-Druckteil verbunden sind. Ein solches System kann die Veränderung der Länge der Verbindungsmittel dadurch vorsehen, dass die Metallschienen in verschiedenen Stellungen am Femur-Druckteil fixiert, beispielsweise verschraubt, werden können. Eine Anpassbarkeit bezüglich der Verbindungsstelle zwischen Beckenring und Verbindungsmitteln ist deshalb zweckmäßig, da bei der Nutzung ein und desselben Beckenrings für Anwender verschiedenen Körperumfangs, der Beckenring in verschiedenen Maße das Becken umfasst. Wird daher bei sehr schlanken Anwendern das Becken fast vollständig umfasst, wird bei korpulenteren Anwendern nur ein Teilbereich, beispielsweise drei Viertel des Beckens umfasst. Die Verstellbarkeit der Verbindungsstelle zwischen Beckenring und Verbindungsmittel erlaubt es, die Verbindungsmittel abhängig von der Statur des Anwenders so zu justieren, dass die Verbindungsmittel sich im Gelenk mit der Gelenkachse des Hüftgelenks kreuzen. Damit Beckenring und Condylenteil bei verschiedenen Anwendern mit verschiedenen Staturen verwendbar sind, sind diese zweckmäßigerweise aus einem flexiblen Material gefertigt, welches ermöglicht, dass Beckenring und Condylenteil jeweils in für die Person des Anwenders sinnvollem Maße zusammengedrückt oder auseinandergedrückt werden können. Dabei bieten sich insbesondere thermoplastische Materialien an. Damit Condylenteil und Beckenring dennoch sicher am Oberschenkel bzw. am Becken Halt finden, sind Verschlussmittel vorgesehen. Bei diesen Verschlussmitteln kann es sich beispielsweise um verstellbare Riemen mit einer Schnalle oder auch um Klettverschlüsse handeln. Die Verschlussmittel sollten so ausgebildet sein, dass sie auch körperlich eingeschränkten Personen eine einfache Verwendung ermöglichen. Bandagenteil, Verschlussriemen, Beckenring und/oder Condylenteil können in verschiedenen Größen zur Verfügung stehen und so ausgebildet sein, dass sie jeweils ein bestimmtes Intervall bzgl. der Körpergröße des Anwenders abdecken.

In einer Weiterbildung der Erfindung ist das Condylenteil um eine bezogen auf den Anwender horizontale Querachse schwenkbar ausgebildet. Dies ermöglicht es, das Condylenteil in Abhängigkeit der Oberschenkelform des Anwenders anzupassen. Auf diese Art und Weise wird ein bequemer und fester Sitz gewährleistet.

Diese und weitere Merkmale von bevorzugten Weiterbildungen der Erfindung gehen außer aus den Ansprüchen auch aus der Beschreibung und den Zeichnungen hervor, wobei die einzelnen Merkmale jeweils für sich allein oder zu mehreren in Form von Unterkombinationen bei einer Ausführungsform der Erfindung und auf anderen Gebieten verwirklicht sein und vorteilhafte sowie für sich schutzfähige Ausführungen darstellen können, für die hier Schutz beansprucht wird. Die Unterteilung der Anmeldung in einzelne Abschnitte sowie Zwischen-Überschriften beschränkt die unter diesen gemachten Aussagen nicht in ihrer Allgemeingültigkeit.

### Kurzbeschreibung der Zeichnungen

Ein Ausführungsbeispiel der Erfindung ist in den folgenden Zeichnungen schematisch dargestellt und wird im folgenden näher erläutert. In den Zeichnungen zeigen:
- Fig. 1: das Orthesenteil einer erfindungsgemäßen Hüftorthese von schräg vorne,
- Fig. 2: das Bandagenteil einer erfindungsgemäßen Hüftorthese von hinten,
- Fig. 3: eine aus Orthesenteil und Bandagenteil zusammengesetzte vollständige erfindungsgemäße Hüftorthese von schräg vorne,
- Fig. 4: verschiedene Stellungen eines Kettengelenks mit zwei Gelenken,
- Fig. 5: verschiedene Stellungen eines Kettengelenks mit drei Gelenken,
- Fig. 6: die maximale Auslenkung eines Kettengelenks nach vorne und nach hinten,
- Fig. 7: einen verstellbaren Verbindungsabschnitt zwischen einem Beckenring und Verbindungsmitteln,
- Fig. 8: den in Fig. 7 dargestellten Verbindungsabschnitt zwischen dem Beckenring und des Verbindungsmitteln in einer geschnittenen Ansicht,
- Fig. 9: eine geschnittene Ansicht von oben auf ein Condylenteil einer erfindungsgemäßen Hüftorthese und
- Fig. 10: das in Fig. 9 dargestellte Condylenteil aus einer seitlichen Perspektive.

### Detaillierte Beschreibung des Ausführungsbeispiels

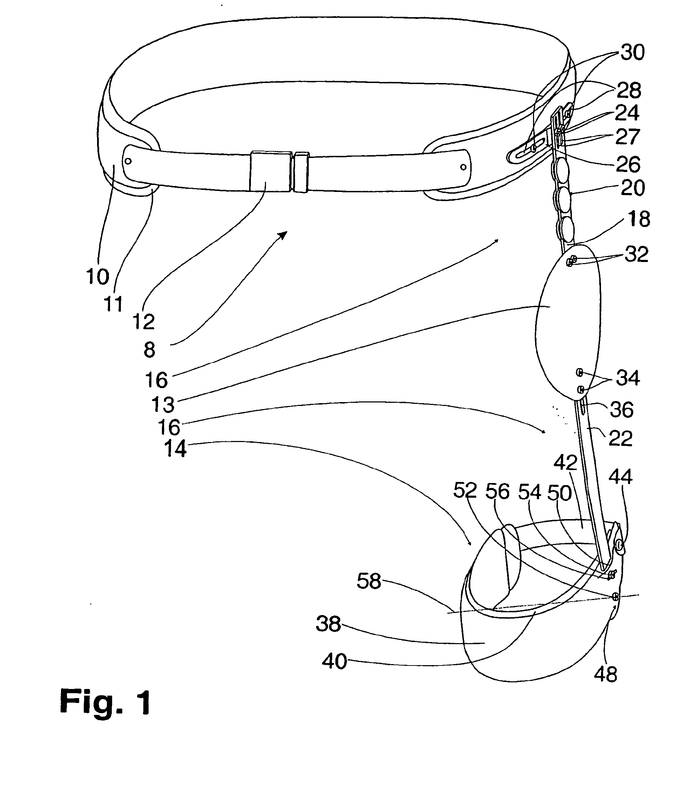

Fig. 1 zeigt einen Orthesenteil 8 einer erfindungsgemäßen Hüftorthese aus einer Perspektive von schräg vorne. Zu erkennen sind dabei der Beckenring 10, die Femur-Druckplatte 13, das Condylenteil 14 sowie die Verbindungsmittel 16. Der Beckenring 10 besteht aus einem flexiblen thermoplastischen Kunststoff und ist so ausgebildet, dass er das Becken eines Anwenders ca. zu drei Vierteln umschließt. Der Beckenring 10 ist mit einem weichen Polster 11 und Verschlussmitteln 12 in Form eines Gurtes mit Schnalle versehen. An der aus der Perspektive von Fig. 1 rechten Seite des Beckenrings 10 ist dieser mit den Verbindungsmitteln 16 verbunden. Die Verbindungsmittel 16 bestehen aus einer oberen Metallschiene 18, die mit einem Kettengelenk 20 aus drei Gelenken versehen ist, und einer unteren Metallschiene 22. Die obere Metallschiene 18 ist mittels zweier Schrauben 24 mit einem Fixierungselement 26 verbunden, das die einstellbare Verbindung mit dem Beckenring 10 ermöglicht. Zu diesem Zweck weist das Fixierungselement 26 zwei horizontale Langlöcher 28 auf, welche ein flexibles Verschrauben des Fixierungselements 26 mittels zweier Schrauben 30 am Beckenring 10 gestatten. Auf diese Art und Weise kann der Schnittpunkt des Beckenrings 10 mit der Metallschiene 18 in der horizontalen Richtung verändert werden. In vertikaler Richtung ist eine Einstellbarkeit durch zwei Langlöcher 27 am oberen Ende der Metallschiene 18 gegeben. Am unteren Ende der oberen Metallschiene 18 ist diese mit der Femur-Druckplatte 13 verbunden. Durch Lösen der Schrauben 24 und Verschieben der Metallschiene 18 relativ zum Beckenring 18 kann der Abstand zwischen dem Beckenring 10 und dem Kettengelenk 20 bzw. der Femur-Druckplatte 13 verändert werden, so dass eine ideal an den Anwender angepasste Einstellung möglich ist. Die Verbindung zwischen der Metallschiene 18 und der Femur-Druckplatte 13 ist dabei mittels zweier Nieten 32 hergestellt. Am unteren Ende der Femur-Druckplatte 13 schließt sich die zweite Metallschiene 22 an. Diese ist mit der Femur-Druckplatte 13 mittels zweier Schrauben 34 verbunden. Die beiden Schrauben 34 sind so angeordnet, dass sie durch ein Langloch 36 in der Metallschiene 22 hindurchragen und auf der gegenüberliegenden Seite auf eine in Fig. 1 nicht erkennbare Art und Weise festgeschraubt sind. Die Länge der Verbindungsmittel 16 kann mittels dieser beiden Schrauben 34 geändert werden. Durch Lösen der Schrauben 34, Verschieben der unteren Metallschiene 22 sowie des Condylenteils 14 und anschließendem Anziehen der Schrauben 34 wird die Gesamtlänge der Verbindungsmittel 16 und damit der Abstand zwischen Beckenring 10 und Condylenteil 14 verändert.

Am unteren Ende der Metallschiene 22 ist diese mit dem Condylenteil 14 verbunden. Das Condylenteil 14 besteht aus einem Kunststoffring 38, der so geartet ist, dass er den Oberschenkel des Anwenders zu ungefähr drei Vierteln umschließt. Auf der Innenseite dieses Kunststoffrings 38 ist eine Polsterung 40. Die Anpassbarkeit an verschiedene Oberschenkelumfänge wird mittels eines Riemens 42 bewerkstelligt, der den Oberschenkel des Anwenders an der hinteren vom Kunststoffring 38 nicht umfassten Seite des Condylenteils 14 umgreift. Der Riemen 42 ist auf einer Seite mittels eines Befestigungsniets 44 mit dem Kunststoffring 38 verbunden. Auf der anderen Seite wird er durch eine Schnalle 46 gehalten, die in der Perspektive der Fig. 1 nicht zu erkennen ist. Die Verbindung zwischen der Metallschiene 22 und dem Condylenteil 14 ist durch Befestigungsmittel 48 gewährleistet, welche eine Einschuböffnung 50 zur Aufnahme der Metallschiene 22 aufweisen. Darüber hinaus weisen die Befestigungsmittel 48 zwei Schrauben 52, 54 auf, die von außen durch die Metallschiene 22 hindurchgeschraubt sind. Die untere dieser Schrauben 52 wird dabei durch eine Bohrung des Befestigungsmittels 48 geführt. Die obere Schraube 54 wird durch ein kreisbogenabschnittsförmiges Langloch 56 geführt. Auf diese Art und Weise ist es möglich, das Condylenteil 14 um die Achse 58 zu kippen, indem die Schraube 54 gelöst, das Condylenteil 14 gekippt und die Schraube 54 anschließend wieder angezogen wird.

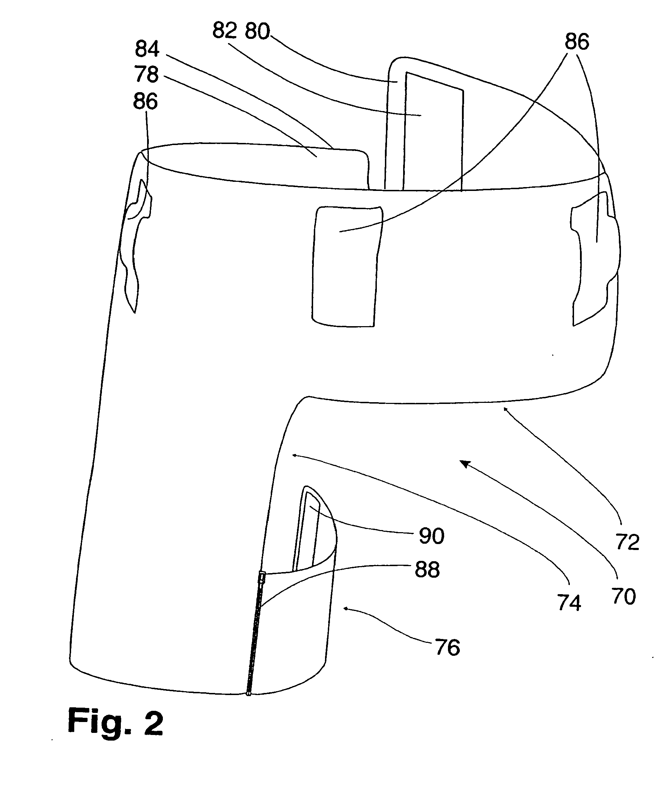

Fig. 2 zeigt einen Bandagenteil 70 einer erfindungsgemäßen Hüftorthese von hinten. Das Bandagenteil 70 verfügt über einen oberen Abschnitt 72, der die Hüfte eines Anwenders umschließt. Ein unterer Abschnitt 74 umschließt gemeinsam mit einem Segment 76 den Oberschenkel des Anwenders. Der obere Abschnitt 72 ist auf der hinteren Seite geschlossen, während er auf der vorderen Seite in zwei Enden 78, 80 mündet, die im Zuge des Anlegens der Orthese miteinander verbunden werden können. Zu diesem Zweck weisen die Enden 78, 80 jeweils einen Klettverschluss 82, 84 auf, wobei in der Perspektive der Fig. 2 nur der Klettverschluss 82 erkennbar ist. Auf der Rückseite des oberen Abschnitts 72 sind Führungsabschnitte 86 vorgesehen, mittels derer das Bandagenteil 70 mit dem Beckenring 10 des Orthesenteils 8 verbunden werden kann.

Das Segment 76 ist mit dem unteren Abschnitt 74 des Bandagenteils 70 auf der hinteren Seite mittels eines Reißverschlusses 88 verbunden. Auf der anderen Seite kann das Segment 76 mit dem unteren Abschnitt 74 des Bandagenteils 70 mittels eines Klettverschlusses 90 verbunden werden. Das Segment 76 ist dementsprechend leicht vom Bandagenteil 70 zu lösen.

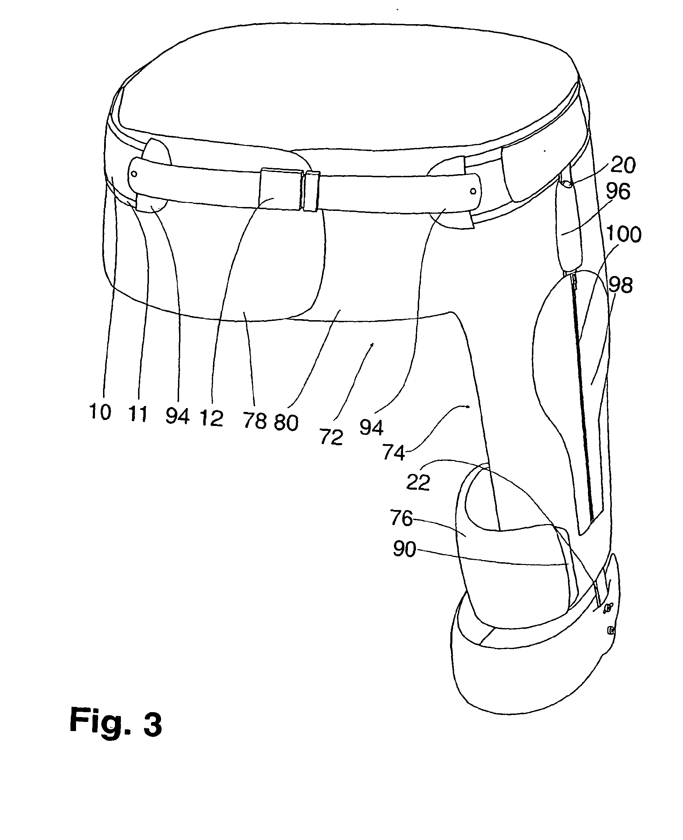

Fig. 3 zeigt die vollständige Hüftorthese aus der Perspektive der Fig. 1 im zusammengesetzten Zustand. Zu erkennen ist, das der Orthesenteil 8 an mehreren Stellen mit dem Bandagenteil 70 verbunden ist. Der Beckenring 10 ist in nicht sichtbarer Art und Weise durch die Befestigungslaschen 86 des Bandagenteils 70 geführt. An den Enden des Polsters 11 ist der Beckenring 10 in dafür vorgesehene Haltetaschen 94 eingesteckt. Über das Kettengelenk 20 ist eine Kettengelenkshülle 96 gestülpt, die das Kettengelenk 20 vor Verschmutzung und mechanischer Beschädigung schützt. Die Femur-Druckplatte 13 ist in eine dafür vorgesehene Tasche 98 eingeführt, welche über einen Reißverschluss 100 verfügt, mittels derer sie geöffnet und geschlossen werden kann. Die Metallschiene 22 ist in dem unteren Abschnitt 74 des Bandagenteils 70 auf der Innenseite des Bandagenteils 70 geführt. Zu erkennen ist darüber hinaus das Segment 76 im geschlossenen Zustand, wobei es auf seiner Vorderseite mit dem unteren Abschnitt 74 des Bandagenteils 70 mittels des Klettverschlusses 90 verbunden ist. Die beiden Enden 78, 80 sind im in Fig. 3 dargestellten Zustand miteinander verbunden. Dies geschieht einerseits mittels des Klettverschlusses 82, 84. Das Orthesenteil 8 wird mittels Verschlussmitteln 12 geschlossen, die bei der dargestellten Ausführungsform als ein Riemen mit einer Schnalle ausgeführt sind.

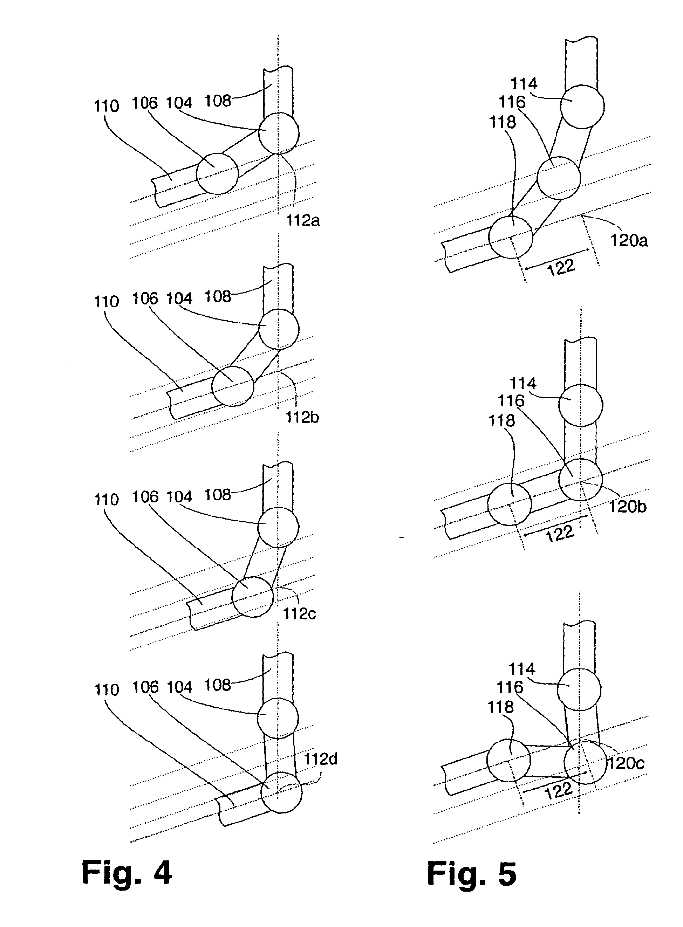

Fig. 4 zeigt die Beweglichkeit eines Kettengelenks mit zwei Gelenken 104, 106, wie es bei einer erfindungsgemäßen Hüftorthese Anwendung findet. Es ist zu erkennen, dass bei gleichbleibender Ausrichtung der Anschlussstücke 108, 110 ein flexibles Einstellen des Schnittpunkts 112a, 122b, 112c, 112d der Anschlusstücke 108, 110 möglich ist. Auf diese Art und Weise erlaubt es ein solches Kettengelenk bestehend aus zwei Gelenken 104, 106, dass sich eine Schwenkachse des Gesamtgelenks durch den Schnittpunkt 112a, 112b, 112c, 112d flexibel so anpasst, dass die Schwenkachse mit der Gelenkachse des Hüftgelenks übereinstimmt. Infolgedessen kommt es nicht zu ungewünschten Kräften, die auf das Hüftgelenk wirken und eine Luxation begünstigen.

Fig. 5 zeigt die Flexibilität eines Kettengelenks mit drei Gelenken 114, 116, 118. Ein solches Kettengelenk mit drei Gelenken 114, 116, 118 ist insofern einem Kettengelenk mit zwei Gelenken 104, 106, wie dem in Fig. 4 dargestellten, überlegen, als dass der Abstand 122 zwischen dem untersten Gelenk 118 und dem Schnittpunkt 120a, 120b, 120c nicht von der Lage des Schnittpunkts zwingend mitbestimmt wird. Dementsprechend unterscheiden sich die verschiedenen Stellungen des Kettengelenks, die in Fig. 5 dargestellt sind, nur hinsichtlich der Lage des Schnittpunktes 120a, 120b, 120c. Der Abstand 122 des letzten Gelenks 118 zum Schnittpunkt 120a, 120b, 120c ist in allen drei Zuständen identisch. Die zwingende Verknüpfung, die, wie in Fig. 4 dargestellt, zwischen der Lage des Schnittpunktes und dem Abstand des letzten Kettengelenks 106 zum Schnittpunkt 112a, 122b, 112c, 112d besteht, wird bei dem in Fig. 5 dargestellten Kettengelenk durch das Vorhandensein eines dritten Gelenks 116 beseitigt. Die Konsequenz daraus ist, dass die Femur-Druckplatte 13 und das Condylenteil 14 des Orthesenteils 8 mit einem solchen Kettengelenk 20 sich beim Beugen des Oberschenkeln nicht mehr relativ zum Oberschenkel bewegen.

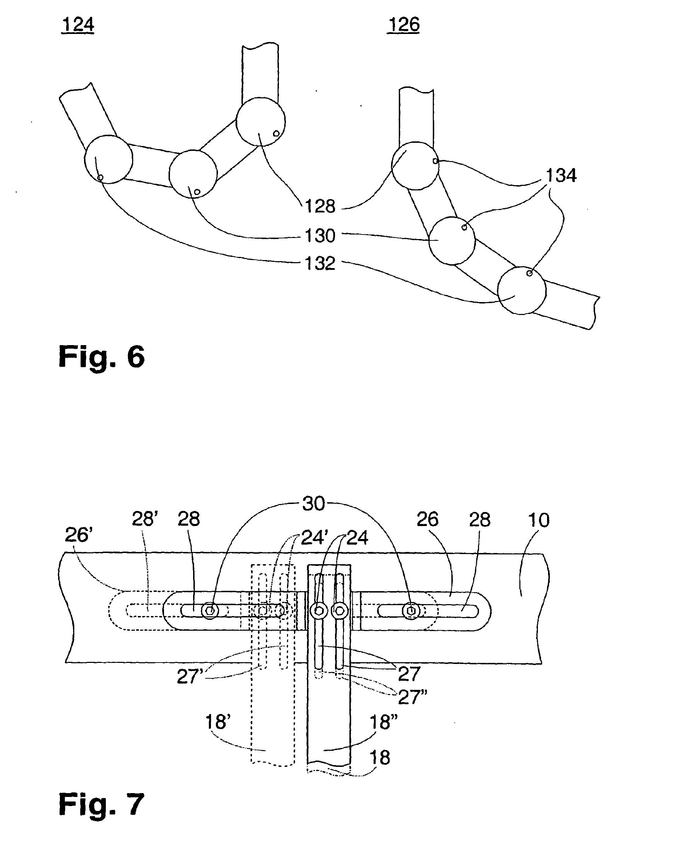

Fig. 6 zeigt zwei Zustände 124, 126 eines Kettengelenks, wobei aus Zustand 124 zu ersehen ist, wie weit das Kettengelenk im maximalen Falle nach hinten ausgelenkt werden kann, während Zustand 126 zeigt, bis zu welchem Grade das Kettengelenk maximal nach vorne ausgelenkt werden kann. Die beim Zustand 124 dargestellte maximale Auslenkung nach hinten beträgt ca. 150°, wobei jedes einzelne Gelenk 128, 130, 132 um jeweils ca. 50° ausgelenkt werden kann. Unter orthopädischen Gesichtspunkten ist die Auslenkung nach hinten kaum erheblich. Eine wesentlich größere Gefahr geht von einer Auslenkung nach vorne aus, da bei einer Auslenkung jenseits von 70° die Gefahr einer Luxation des Hüftgelenks deutlich erhöht wird und eine solche Oberschenkelstellung, beispielsweise beim Sitzen, üblich ist. Daher ist durch das Kettengelenk im Zustand 126 die maximale Auslenkung auf ca. 70° begrenzt, wobei jedes der Einzelgelenke 128, 130, 132 jeweils eine Auslenkung von ca. 23° zulässt. Dadurch wird die Bewegungsfreiheit des Anwenders in einer unter orthopädischen Gesichtspunkten sinnvollen Art und Weise beschränkt. Die Beschränkung des jeweiligen Winkels wird beim dargestellten Ausführungsbeispiel über kleine Metallstifte 134 realisiert. Die Metallstifte 134 sind Teil der Gelenke. Beim jeweils vorgegebenen Winkel stößt die Metallschiene gegen einen jeweiligen Metallstift 134 und wird dadurch von einer weiteren Drehung gegenüber dem Gelenk abgehalten.

Fig. 7 zeigt das Fixierungselement 26, mit dem die Metallschiene 18 mit dem Beckenring 10 verbunden ist. Das Fixierungselement 26 ist dabei mittels zweier Schrauben 24 mit der Metallschiene 18 verbunden. Im Befestigungsmittel 26 sind zwei Langlöcher 28 vorgesehen, die es erlauben, die Kombination aus Metallschiene 18 und Fixierungselement 26 an einer individuell angepassten Stelle des Beckenrings 10 zu befestigen. Dies geschieht mittels der Schrauben 30, die nach Zurechtschieben des Fixierungselements 26 in die angepasste Lage angezogen werden. Zur Verdeutlichung zeigen gestrichelt dargestellt die Metallschiene 18', das Fixierungselement 26' mit den beiden Langlöchern 28', wie die Stellung des Fixierungselements 26 und der Metallschiene 18 dem Beckenring 10 gegenüber verändert werden kann. Die Metallschiene 18 weist ebenso zwei Langlöcher 27 auf, die eine vertikale Einstellung ermöglichen. Durch das Lösen der Schrauben 24, das Verschieben der Metallschiene 18 relativ zum Beckenring 10 und das anschließende WiederAnziehen der beiden Schrauben 24 kann das Kettengelenk in der Höhe optimal auf die anatomischen Gegebenheiten des Anwenders eingestellt werden. Die vertikale Einstellbarkeit ist anhand der strichpunktiert dargestellten verschobenen Metallschiene 18" verdeutlicht.

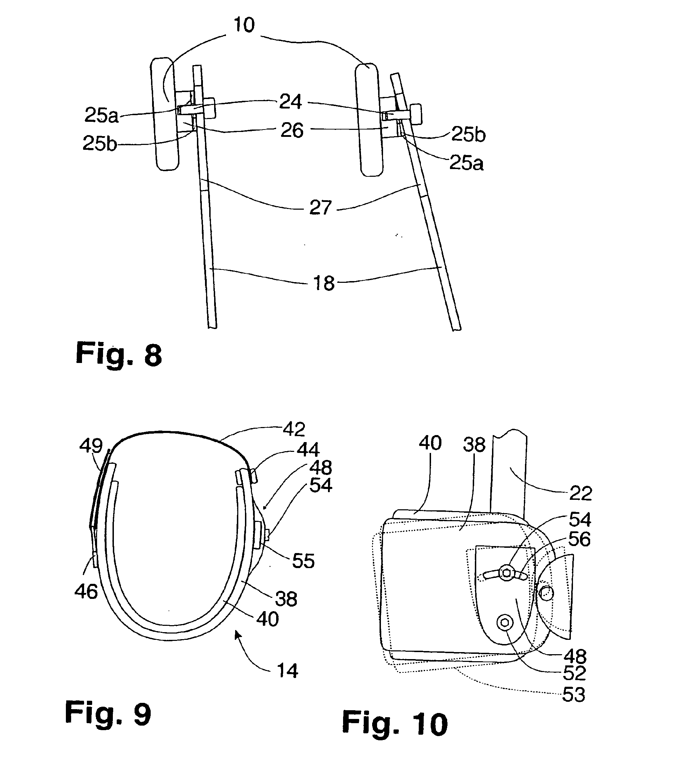

Fig. 8 zeigt das in Fig. 7 dargestellte Fixierungselement 26, die Metallschiene 18 und den Beckenring 10 in einer geschnittenen Ansicht. Zu erkennen ist, dass zwischen der Metallschiene 18 und dem Fixierungselement 26 zwei Keilelemente 25a, 25b vorgesehen sind, mittels derer der Abduktionswinkel der Metallschiene 18 eingestellt werden kann. Die linke Darstellung zeigt eine Einstellung, bei der die spitzen Winkel beider Keilelemente 25a, 25b in entgegengesetzt Richtung weisen. Dadurch hebt sich ihre Wirkung gegenseitig auf und der Abduktionswinkel der Metallschiene 18 wird einzig durch den Beckenring 10 bestimmt. Bei der rechten Darstellung weist der spitze Winkel beider Keilelemente 25a, 25b nach oben, so dass sie jeweils zu einer Vergrößerung des Abduktionswinkels, also dem Winkel zwischen einer Senkrechten und der Metallschiene, beitragen. Eine feinere Abstimmung ist durch die Verwendung anderer Keilelemente möglich, so dass eine orthopädisch gut auf den Anwender abgestimmte Abduktion erreicht werden kann.

Fig. 9 zeigt das Condylenteil 14 in einer Ansicht von oben. Zu erkennen sind dabei die Befestigungsmittel 48 mit der Schraube 54 sowie einer Aussparung 55, in die in montiertem Zustand die Metallschiene 22 eingeführt ist. Das wesentliche Element des Condylenteils 14 ist der Kunststoffring 38. Er umfasst den Oberschenkel des Anwenders vorne und seitlich. Für einen bequemeren Sitz ist ein Polster 40 vorgesehen, welches mit dem Kunststoffring 38 verbunden ist, beispielsweise mit einem Klettverschluss oder geklebt. Der Kunststoffring 38 ist so dimensioniert, dass er auch bei einem schmalen Oberschenkel diesen nicht vollständig umgreift. Zur Anpassung des Condylenteils 14 an verschiedene Anwender ist ein Riemen 42 vorgesehen, der an einer Seite mit einem Befestigungsniet 44 mit dem Kunststoffring 38 verbunden ist. Auf der anderen Seite des Kunststoffrings 38 ist eine Halteeinrichtung 46 vorgesehen, durch die der Riemen gezogen wird, so dass das Condylenteil 14 fest um den Oberschenkel des Anwenders anliegt. Die Fixierung des Riemens 42 kann beispielsweise über einen Klettverschluss 49 erfolgen.

Fig. 10 zeigt das in Fig. 9 dargestellte Condylenteil 14 aus einer seitlichen Perspektive. Zu erkennen ist der Kippmechanismus, der aus den beiden Schrauben 52, 54 sowie dem gebogenen Langloch 56 besteht. Durch Lösen beider Schrauben 52, 54 und anschließendem Kippen des Condylenteils 14 gegenüber der Metallschiene 22 kann eine individuelle Einstellung des Kippwinkels an den Anwender erfolgen. Die gestrichelte Darstellung 53 zeigt ein Condylenteil in einem gegenüber der Metallschiene 22 gekippten Zustand.

## Patentansprüche

1. Hüftorthese zum Anlegen im Bereich der Hüfte und eines Oberschenkels eines Anwenders mit einem Orthesenteil (8), bestehend aus einem Beckenring (10), einem Femur-Teil (13), einem Condylenteil (14) und Verbindungsmitteln (16) mit einem Gelenk (20), wobei die Verbindungsmittel (16) den Beckenring (10) mit dem Condylenteil (14) verbinden, wobei das Femur-Teil (13) entweder zwischen dem Beckenring (10) und dem Condylenteil (14) mit den Verbindungsmitteln (16) verbunden ist oder ein Bestandteil der Verbindungsmittel (16) ist, und wobei das Gelenk (20) ein Beugen des Oberschenkels ermöglicht,
**dadurch gekennzeichnet, dass**
das Gelenk (20) mehrere Gelenkachsen aufweist, die gemeinsam eine Bewegungsfläche für eine Bewegung des Condylenteils (14) und des Femur-Teils (13) relativ zum Beckenring definieren, wobei das Gelenk (20) in eine Richtung senkrecht zur Bewegungsfläche beziehungsweise parallel zu einer Gelenkachse federnd ausgebildet ist.

2. Hüftorthese nach Anspruch 1,
**dadurch gekennzeichnet, dass**
das Gelenk (20) ein Kettengelenk mit parallelen Gelenkachsen ist, wobei die Anzahl der Achsen mindestens zwei und vorzugsweise drei ist.

3. Hüftorthese nach Anspruch 1 oder 2,
**dadurch gekennzeichnet, dass**
das Gelenk (20) ein Beugen des Oberschenkels in einer oder beiden Richtungen bei einem jeweils festgelegten Winkel begrenzt, nach vorne vorzugsweise bei ca. 60° bis 80°, insbesondere 70°.

4. Hüftorthese nach Anspruch 1,
**gekennzeichnet durch**
ein Bandagenteil (70), das mit dem Orthesenteil (8) verbunden ist, vorzugsweise lösbar, und das so ausgebildet ist, dass es zumindest einen Teil der Hüfte und des Oberschenkels umschließt.

5. Hüftorthese nach Anspruch 4,
**dadurch gekennzeichnet, dass**
ein Oberschenkelsegment (76) des Bandagenteils (70), insbesondere ein Oberschenkelsegment (76) an der Innenseite des Oberschenkels, lösbar mit der übrigen Bandage (72, 74) verbunden ist, vorzugsweise mittels eines Reißverschlusses (88) und/oder eines Klettverschlusses (90).

6. Hüftorthese nach Anspruch 1 oder 4,
**gekennzeichnet durch**
eine Anpassbarkeit an verschiedene Anwender mit verschiedenen Körpermaßen derart, dass die Verbindungsmittel (16) bezüglich ihrer Länge änderbar sind, dass die Verbindungsmittel (16) bezüglich ihrer Verbindungsstelle mit dem Beckenring (10) verstellbar sind und/oder dass der Beckenring (10) und das Condylenteil (14) aus flexiblem Material sind und mittels elastischem und/oder verstellbarem Verschlussmitteln (82, 84, 102) an verschiedene Becken- bzw. Knieumfänge anpassbar sind.

7. Hüftorthese nach Anspruch 6,
**dadurch gekennzeichnet, dass**
das Condylenteil (14) um eine bezogen auf den Anwender horizontale Querachse (58) schwenkbar ausgebildet ist.

## Claims

1. Hip orthosis for application in the area of the hips and thigh of a user having an orthosis part (8) comprising a pelvic girdle (10), a femur part (13), a condyle part (14) and connecting means (16) with a joint (20), the connecting means (16) connecting the pelvic girdle (10) to the condyle part (14), the femur part (13) either being connected between pelvic girdle (10) and condyle part (14) to the connecting means (16) or forms part of the connecting means (16), and in which the joint (20) permits a bending of the thigh, **characterized in that** the joint (20) has several joint axes, which jointly define a movement area for a movement of condyle part (14) and femur part (13) relative to the pelvic girdle, the joint (20) being resiliently constructed in a direction perpendicular to the movement area or parallel to a joint axis.

2. Hip orthosis according to claim 1, **characterized in that** joint (20) is a chain joint with parallel joint axes, there being at least two and preferably three axes.

3. Hip orthosis according to claim 1 or 2, **characterized in that** joint (20) limits a bending of the thigh in one or both directions at an in each case fixed angle in the forwards direction preferably of approximately 60 to 80°, particularly 70°.

4. Hip orthosis according to claim 1, **characterized in that** a bandage part (70) connected to orthosis part (8) is preferably detachable and constructed in such a way that it surrounds at least part of the hips and thigh.

5. Hip orthosis according to claim 4, **characterized in that** a thigh segment (76) of bandage part (70), particularly a thigh segment (76) on the inside of the thigh, is detachably connected to the remaining bandage (72, 74), preferably by means of a zip fastener (88) and/or a Velcro fastener (90).

6. Hip orthosis according to claims 1 or 4, **characterized by** an adaptability to different users with different body sizes in such a way that the length of the connecting means (16) can be varied, that the connecting means (16) can be adjusted with respect to their connection point to the pelvic girdle (10) and/or that the pelvic girdle (10) and condyle part (14) are made from flexible material and by means of elastic and/or adjustable closure means (82, 84, 102) can be adapted to different pelvis or knee circumferences.

7. Hip orthosis according to claim 6, **characterized in that** the condyle part (14) is pivotable about a transverse axis (58) horizontal relative to the user.

## Revendications

1. Orthèse de la hanche pour un endossement dans le domaine de la hanche et d'une cuisse d'un utilisateur, avec un élément d'orthèse (8) composé d'une ceinture du bassin (10), d'un élément fémoral (13), d'un élément condylien (14) et de pièces de raccordement (16) avec une articulation (20), sachant que les pièces de raccordement (16) raccordent la ceinture du bassin (10) à l'élément condylien (14), et sachant que l'élément fémoral (13) est soit raccordé entre la ceinture du bassin (10) et l'élément condylien (14) avec les pièces de raccordement (16) soit qu'il fait partie des pièces de raccordement (16) et sachant que l'articulation (20) permet de fléchir la cuisse,
**caractérisé en ce que**
l'articulation (20) présente plusieurs axes d'articulation, qui définissent ensemble un plan de mouvement pour un mouvement de l'élément condylien (14) et de l'élément fémoral (13) par rapport à la ceinture du bassin, sachant que l'articulation (20) est réalisée de manière élastique à ressort dans une direction orthogonale par rapport au plan de mouvement ou encore parallèle par rapport à un axe d'articulation.

2. Orthèse de la hanche d'après la revendication 1,
**caractérisée en ce que**
l'articulation (20) est une articulation à chaîne ayant des axes d'articulation parallèles, sachant que le nombre d'axes s'élève au moins à deux et de préférence à trois.

3. Orthèse de la hanche d'après la revendication 1 ou 2,
**caractérisée en ce que** l'articulation (20) limite un fléchissement de la cuisse en une ou dans les deux directions respectivement pour un angle bien déterminé, en avant de préférence à un angle approximatif entre 60° et 80° et notamment de 70°.

4. Orthèse de la hanche d'après la revendication 1,
**caractérisée par**
un élément à bandage (70) raccordé à l'élément d'orthèse (8), préférablement de manière détachable, et qui est réalisé de manière qu'il entoure au moins une partie de la hanche et de la cuisse.

5. Orthèse de la hanche d'après la revendication 4,
**caractérisée en ce qu'**un
segment fémoral (76) de l'élément à bandage (70), notamment un segment fémoral (76) à la face intérieure de la cuisse est raccordé de manière détachable au reste du bandage (72, 74), de préférence au moyen d'une fermeture-éclair (88) et/ou d'un ruban autoaccrochant (90).

6. Orthèse de la hanche d'après la revendication 1 ou 4,
**caractérisée par**
une adaptabilité à des utilisateurs de tailles différentes, de manière que les moyens de raccordement (16) soient modifiables dans leur longueur, de manière que les moyens de raccordement (16) soient ajustables par rapport à leur endroit de raccordement avec la ceinture du bassin (10) et/ou de manière que la ceinture du bassin (10) et l'élément condylien (14) sont réalisés en un matériau flexible et qu'ils peuvent être adaptés à différentes circonférences du bassin et du genou par des dispositifs de fermeture (82, 84, 102) ajustables.

7. Orthèse de la hanche d'après la revendication 6,
**caractérisée en ce que**
l'élément condylien (14) est réalisé de manière à être pivotable autour d'un axe transversal (58), horizontal par rapport à l'utilisateur.
